# EUROPÄISCHE PATENTSCHRIFT

(11) **EP 0 727 972 B1**
(45) Veröffentlichungstag und Bekanntmachung des Hinweises auf die Patenterteilung: **12.03.1997**
(21) Anmeldenummer: 95900691.7
(22) Anmeldetag: 09.11.1994
(51) Int. Cl.: A61F 9/08, G09B 21/00, A61F 4/00

(54) **ANORDNUNG ZUR BILDERKENNUNG FÜR BLINDE UND HOCHGRADIG SEHBEHINDERTE**
IMAGE RECOGNITION DEVICE FOR BLIND AND SEVERELY VISUALLY HANDICAPPED PERSONS
DISPOSITIF DE RECONNAISSANCE D'IMAGE POUR AVEUGLES ET PERSONNES AYANT UNE TRES MAUVAISE VUE

(30) Priorität: 12.11.1993 DE 4339237
(43) Veröffentlichungstag der Anmeldung: 28.08.1996
(73) Patentinhaber: Schimmelpfennig, Winfried, D-18292 Krakow am See (DE)
(72) Erfinder: Schimmelpfennig, Winfried, D-18292 Krakow am See (DE)
(74) Vertreter: Schnick, Achim
(86) Internationale Anmeldenummer: EP9403704
(87) Internationale Veröffentlichungsnummer: WO9513037

(56) Entgegenhaltungen:
- EP-A- 0 487 027
- WO-A-93/03468
- DE-A- 1 762 806
- DE-C- 326 283
- DE-C- 1 274 836
- FR-A- 2 598 316
- US-A- 3 612 061

## Beschreibung

Die Erfindung betrifft eine Anordnung zur Bilderkennung für Blinde und hochgradig Sehbehinderte mit einer Einrichtung, die die Bildinformation auf nicht-optischem Wege darstellt.

Gemäß dem bekannten Stand der Technik, der durch die DE-OS 23 30 403 dokumentiert wird, gibt es Sehhilfen für Blinde, die mit Hilfe einer Elektroden-Matrix elektrische Potentialbilder direkt von der Bildquelle auf die Haut der Stirn oder eines Fingers projizieren. Da der elektrische Hautwiderstand überwunden werden muß, sind an diesen Elektroden erhebliche langzeitschädliche elektrische Spannungen nötig, es treten unerwünschte galvanische und thermische Nebeneffekte auf, die räumliche Auflösung ist sehr gering, so daß von einer wirklichen Bilderkennung nicht die Rede sein kann.

Aus dem Jahre 1920 ist durch die DE-C-326 283 auch schon eine Vorrichtung bekannt, die die Zunge zur Bilderkennung nutzt. Bei dieser Vorrichtung erfolgt eine direkte Kopplung von Empfangssensoren, in diesem Patent eine Selenstruktur, mit einer geometrisch gleichartigen Elektrodenmatrix im Zungenbereich, und zwar Bildpunkt für Bildpunkt einzeln direkt kontaktiert auf die entsprechende Elektrode im Zungenbereich. Den für einen Stromfluß nötigen Gegenpol stellt die Zunge selbst dar, die von den Lippen her großflächig kontaktiert ist. Durch dieses Verfahren ist die Einbeziehung des gesamten Mundbereiches in den Stromfluß gegeben, was physiologisch bedenklich ist. Außerdem ist durch die direkte Kopplung der Selensensoren mit den Elektroden keine zwischengeschaltete Bildbearbeitung möglich, die die Bilderkennung wesentlich verbessern kann.

Aufgabe der Erfindung ist es, einem Blinden oder hochgradig Sehbehinderten eine Hilfe für die Bilderkennung und Orientierung in seiner Umgebung bereitzustellen, indem seine eingeschränkte optische Wahrnehmungsfähigkeit durch technische Hilfsmittel im Zusammenwirken mit anderen, funktionsfähigen Sinnesorganen des Behinderten kompensiert wird.

Erfindungsgemäß wird diese Aufgabe dadurch gelöst, daß die Signale zur Darstellung der Bildinformation, die elektronisch aufbereitet sind, auf eine im Mundraum im Tastbereich der Zunge tragbare flache Monitor-Matrix übertragen werden. Nach der Erfindung werden als Darstellungsprinzipien Potential-Verteilungsbilder, Temperatur-Verteilungsbilder oder Reliefbilder angewandt.

Zur Erzeugung eines elektrischen Stromdichte-Bildes bestehen die Aktoren der Matrix aus Elektrodenpaaren, die zu Zeilen- und Spaltenleitungen durchkontaktiert sind, wobei jeder Bildpunkt dem lokalen Stromfluß innerhalb eines einzelnen Elektrodenpaares zugeordnet ist.

Im Falle der Anwendung eines Temperaturbildes bestehen die Aktoren der Matrix aus quasi-punktförmigen elektrischen Heizelementen, die in den Knotenpunkten zwischen den Spalten- und Zeilensignalen der Matrix kontaktiert sind.

Die Aktoren der Matrix zur erfindungsgemäßen Erzeugung eines Relief-Bildes bestehen aus in Zeilen und Spalten angeordneten mikromechanischen Bildpunkt-Elementen.

Nach einem weiteren Merkmal der Erfindung weist die Monitor-Matrix Sensoren auf, die die Position der Zungenspitze zur Steuerung von Computerparametern erfassen.

Ausgehend vom Signalfluß wird in der Praxis durch einen systematischen modularen Aufbau des Systems, von der Bildaufnahme über die computertechnische Bildbearbeitung, Stabilisierung, Filterung und Kontrastverstärkung bis zur Signalumsetzung und Darstellung auf der Matrix, eine stetige Vervollkommnung und Erweiterung der Anwendungsfelder des Systems durch Austausch und Optimierung der einzelnen Module erreicht.

Gegenüber dem bekannten Stand der Technik bringt die erfindungsgemäße Bilderkennung über die Zunge erstmals die Möglichkeit, sich auf einer speziell für diesen Anwendungszweck entwickelten Matrix mit vielen tausenden Bildpunkten über die Zungenspitze auf ein beliebiges Bild-Detail zu konzentrieren und diesen Teil der Matrix genauer abzutasten und den Bildinhalt zu analysieren. Dies entspricht beim Sehenden dem Prinzip des "Hinsehens" des Auges, das ja ebenfalls nur über einen kleinen Bereich des scharfen Sehens verfügt. Dadurch die für den Blinden im gesamten Matrixbereich eine ganz wesentliche Erhöhung der räumlichen Bildauflösung gegeben.

Da die sehr empfindliche und hochauflösende Zungenspitze zur Bildabtastung verwendet wird, ist der nötige Energiebedarf der gesamten Matrix um Größenordnungen geringer, damit auch alle schädlichen Nebeneffekte und die Abmessungen sind trotz der relativ hohen Anzahl der Bildpunkte so klein, daß die Matrix unauffällig wie eine Zahnprothese getragen werden kann.

Die erfindungsgemäße Matrix zur Potentialbild-Darstellung hat beispielsweise eine Höhe von ca. 20 mm und eine Breite von ca. 40 mm. Ihre Fläche ist der Gaumenform des Benutzers angepaßt und bildet die Matrix aus veredelten Elektrodenpaaren, die in Spalten und Zeilen kontaktiert sind. Die gesamte Matrix ist oberflächenpoliert, so daß die Zunge die Anordnung der Elektrodenpaare mechanisch nicht spüren kann, also nicht irritiert wird.

Wird nun ein elektrisches Stromdichtebild auf die Elektrodenpaare projiziert, so wirkt dadurch jeder einzelne Bildpunkt für die Zunge wie ein winziges mechanisches Hindernis, das sich nicht wegwischen läßt; es entsteht so der gefühlsmäßige Eindruck eines Reliefbildes, das mit der Zungenfläche großflächig und mit der Zungenspitze im Detail abgefühlt wird. Galvanische Effekte werden durch eine spezielle potentialfreie Wechsel-Signalform vollständig unterdrückt. Durch die Ansteuerung von Elektrodenpaaren wird bei jedem Bildpunkt nur ein lokal begrenzter kurzer Stromweg generiert, die Gesamtmatrix bleibt gegebenüber dem Mundbereich elektrisch neutral.

## Patentansprüche

1. Anordnung zur Bilderkennung für Blinde und hochgradig Sehbehinderte mit einer in den Mundbereich einsetzbaren Einrichtung, die die Bildinformation auf nicht-optischem Wege darstellt und die durch die Zunge abgefühlt werden kann, dadurch gekennzeichnet, daß die Signale zur Darstellung der Bildinformation auf eine im Mundraum im Tastbereich der Zunge tragbare flache Monitor-Matrix übertragen werden.

2. Anordnung zur Bilderkennung nach Anspruch 1, dadurch gekennzeichnet, daß die auf die Monitor-Matrix übertragenen Signale elektronisch aufbereitet sind.

3. Anordnung zur Bilderkennung nach den Ansprüchen 1 und 2, dadurch gekennzeichnet, daß die Aktoren zur Erzeugung eines elektrischen Stromdichte-Bildes aus Elektrodenpaaren bestehen, die zu Zeilen- und Spaltenleitungen durchkontaktiert sind, wobei jeder Bildpunkt dem lokalen Stromfluß innerhalb eines einzelnen Elektrodenpaares zugeordnet ist.

4. Anordnung zur Bilderkennung nach den Ansprüchen 1 und 2, dadurch gekennzeichnet, daß die Aktoren zur Erzeugung eines Temperatur-Bildes aus quasi-punktförmigen elektrischen Heizelementen bestehen, die in den Knotenpunkten zwischen den Spalten- und Zeilensignalen der Matrix kontaktiert sind.

5. Anordnung zur Bilderkennung nach den Ansprüchen 1 und 2, dadurch gekennzeichnet, daß die Aktoren zur Erzeugung eines Relief-Bildes aus in Zeilen und Spalten angeordneten mikromechanischen Bildpunkt-Elementen bestehen.

6. Anordnung zur Bilderkennung nach den Ansprüchen 1 bis 5, dadurch gekennzeichnet, daß die Monitor-Matrix Sensoren auf weist, die die Position der Zungenspitze zur Steuerung von Computerparametern erfassen.

## Claims

1. Image recognition device for blind and severely visually handicapped persons with a device which can be inserted into the mouth area which represents the visual information in an non-optical way and which can be felt by the tongue, characterised in that the signals for representing the visual information are transferred to a flat monitor matrix which can be carried in the mouth in the region of the tongue.

2. Image recognition device according to claim 1, characterised in that the signals transmitted to the monitor matrix are edited electronically.

3. Image recognition device according to claims 1 and 2, characterised in that the actuators for generating an electric current density image comprise electrode pairs which are plated through to row and column lines whereby each image point is assigned to the local current flow within an individual electrode pair.

4. Image recognition device according to claims 1 and 2, characterised in that the actuators for generating a temperature image comprise almost point-like electric heating elements which are contacted in the nodal points between the column and row signals of the matrix.

5. Image recognition device according to claims 1 and 2, characterised in that the actuators for generating a relief image comprise micromechanical image point elements arranged in rows and columns.

6. Image recognition device according to claims 1 to 5, characterised in that the monitor matrix comprises sensors which detect the position of the tip of the tongue to control computer parameters.

## Revendications

1. Dispositif destiné à l'identification d'images pour aveugles et personnes souffrant d'un handicap visuel profond, comportant un appareil pouvant être placé dans la région buccale et qui restitue l'information d'image par des voies non optiques et pouvant être captée par la langue, caractérisé en ce que les signaux pour la restitution de l'information d'image sont transmis sur une matrice de moniteur plat pouvant être fixée dans la cavité buccale dans la zone de contact avec la langue.

2. Dispositif destiné à l'identification d'images selon la revendication 1, caractérisé en ce qu'il prépare électroniquement les signaux transmis sur la matrice de moniteur.

3. Dispositif destiné à l'identification d'images selon les revendications 1 et 2, caractérisé en ce que les acteurs pour la génération d'une image de densité électrique consistent en des paires d'électrodes qui sont contactées en conduite de lignes et de colonnes, chaque point d'image étant affecté au flux de courant local à l'intérieur d'une seule paire d'électrodes.

4. Dispositif destiné à l'identification d'images selon les revendications 1 et 2, caractérisé en ce que les acteurs pour la génération d'une image de température sont constitués par des éléments de chauffage électrique quasi-ponctuels qui sont contactés aux points nodaux entre les signaux de colonnes et de lignes de la matrice.

5. Dispositif destiné à l'identification d'image selon les revendications 1 et 2, caractérisé en ce que les acteurs pour la génération d'une image en relief sont constitués par des éléments d'image mécaniques disposés en lignes et en colonnes.

6. Dispositif destiné à l'identification d'image selon les revendications 1 à 5, caractérisé en ce que la matrice de moniteur comporte des capteurs qui saisissent la position de la pointe de la langue pour commander les paramètres d ordinateur.
